(19) Europäisches Patentamt
European Patent Office
Office européen des brevets

(11) **EP 4 410 418 A1**

(12) **EUROPEAN PATENT APPLICATION**
published in accordance with Art. 153(4) EPC

(43) Date of publication:
**07.08.2024 Bulletin 2024/32**

(21) Application number: **22875883.5**

(22) Date of filing: **16.09.2022**

(51) International Patent Classification (IPC):
**B01J 20/22** (2006.01)     **A61L 9/014** (2006.01)
**B01D 39/14** (2006.01)     **B01J 20/28** (2006.01)
**C01B 32/354** (2017.01)     **C01B 33/18** (2006.01)

(52) Cooperative Patent Classification (CPC):
**A61L 9/014; B01D 39/14; B01J 20/22; B01J 20/28;
C01B 32/354; C01B 33/18**

(86) International application number:
**PCT/JP2022/034719**

(87) International publication number:
**WO 2023/054012 (06.04.2023 Gazette 2023/14)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.09.2021  JP 2021160477**

(71) Applicant: **Toray Industries, Inc.
Tokyo 103-8666 (JP)**

(72) Inventors:
• **YAMAGA, Naoki**
 **Otsu-shi, Shiga 520-8558 (JP)**
• **HAYASHI, Toshiki**
 **Otsu-shi, Shiga 520-8558 (JP)**
• **RAKUMA, Kento**
 **Otsu-shi, Shiga 520-8558 (JP)**

(74) Representative: **Hoefer & Partner Patentanwälte
mbB
Pilgersheimer Straße 20
81543 München (DE)**

(54) **GAS ADSORBENT AND GAS ADSORPTION SHEET, FILTER MEDIUM, AND AIR FILTER USING SAME**

(57)     An object of the present invention is to obtain a gas adsorbent, a gas adsorption sheet, a filter medium, and an air filter that are excellent in gas component adsorption capacity and excellent in long-term stability of the adsorption capacity and also re-release little odor after being used as an air filter. A main object of the present invention is to provide a gas adsorbent that contains an acid-impregnated activated carbon, a base-impregnated activated carbon, and an aldehyde adsorbent.

**EP 4 410 418 A1**

**Description**

TECHNICAL FIELD

The present invention relates to a gas adsorbent, and a gas adsorption sheet, a filter medium, and an air filter using the gas adsorbent.

BACKGROUND ART

Air purifiers are used for the purpose of removing dust and odor gas components in homes. In recent years, as required performance for a deodorizing function of a household air purifier, deodorizing performance for volatile organic compounds (VOC) generated in a living environment where the air purifier is used is required. Among them, VOC such as formaldehyde has been shown to have an undesirable influence on the human body, and it is prescribed by the laws and regulations of each country to control the indoor environmental concentration to a certain level or less. The air purifier is expected to have performance as a removal device for this VOC. In addition, there is a demand for high removal efficiency for formaldehyde, which is considered to be a cause of sick house syndrome and is at risk of being released from b uilding materials such as wallpaper, and for long life of the removal performance. The high deodorizing efficiency indicates that the operating time of the air purifier required to lower the gas concentration in a predetermined space is short. The long life means that the deodorizing efficiency is maintained even after a predetermined amount of gas is adsorbed. The deodorizing efficiency is indicated by a clean air delivery rate (CADR), and the life is indicated by a cumulate clean mass (CCM) as evaluation standards in GB/T 18801 2015 (China National Standard) or the like.

In order to satisfy these required performances, as an air filter used for an air purifier, an air filter supporting a mixture of activated carbon and an adsorbent which adsorbs aldehydes by chemical reactivity has been proposed (see, for example, Patent Document 1).

In addition, as a method for suppressing a phenomenon (secondary odor generation) of re-releasing adsorbed gas during use as a filter, a filter medium including an alkaline deodorizer disposed on an air outflow side and a neutral or/and acidic deodorizer disposed on an air inflow side has been proposed (see, for example, Patent Document 2) .

PRIOR ART DOCUMENT

PATENT DOCUMENTS

Patent Document 1: Japanese Patent Laid-open Publication No. 2008-148804
Patent Document 2: Japanese Patent Laid-open Publication No. 2016-171875

SUMMARY OF THE INVENTION

PROBLEMS TO BE SOLVED BY THE INVENTION

However, according to findings by the inventors, an air filter actually obtained based on the design concept described above has a problem that odor components adsorbed to the air filter are desorbed from the air filter and released into a space (secondary odor generation).

In view of the above circumstances, the present invention provides an adsorbent capable of maintaining the ability to remove odor components such as formaldehyde for a long period of time, and suppressing desorption of adsorbed gas components from an air filter by use, that is, effective for suppressing secondary odor generation.

SOLUTIONS TO THE PROBLEMS

The gas adsorbent of the present invention for solving the above problems contains an acid-impregnated activated carbon (A1), a base-impregnated activated carbon (A2), and an aldehyde adsorbent (B).

The gas adsorbent of the present invention preferably satisfies the following (1) to (4).

(1) The ratio $(M_A) : (M_B)$ of the total mass $(M_A)$ of the acid-impregnated activated carbon (A1) and the base-impregnated activated carbon (A2) to the mass $(M_B)$ of the aldehyde adsorbent (B) is 95 : 5 to 25 : 75.
(2) The average particle diameter of the acid-impregnated activated carbon (A1) is 40 to 500 um, the average particle diameter of the base-impregnated activated carbon (A2) is 40 to 500 um, and when the average particle diameter of the aldehyde adsorbent (B) is 100, the average particle diameter of the acid-impregnated activated carbon (A1) is 95 to 600, and the average particle diameter of the base-impregnated activated carbon (A2) is 95 to 600.

(3) The ratio of the pore volume of pores having a pore diameter of 0.4 to 2.0 nm of the acid-impregnated activated carbon (A1), calculated by the MP method to the pore volume of pores having a pore diameter of 0.4 nm or more of the acid-impregnated activated carbon (A1), calculated by the MP method and the BJH method is 75 to 100%, and/or the ratio of the pore volume of pores having a pore diameter of 0.4 to 2.0 nm of the base-impregnated activated carbon (A2), calculated by the MP method to the pore volume of pores having a pore diameter of 0.4 nm or more of the base-impregnated activated carbon (A2), calculated by the MP method and the BJH method is 75 to 100%.

(4) The specific surface area of the acid-impregnated activated carbon (A1) and/or the base-impregnated activated carbon (A2) is 400 to 1,300 $m^2/g$.

The gas adsorption sheet of the present invention contains the gas adsorbent of the present invention.

In the filter medium of the present invention, the gas adsorbent of the present invention is held in at least one interface among one or more interfaces formed by two or more layers of nonwoven fabric.

The air filter of the present invention includes the filter medium of the present invention.

EFFECTS OF THE INVENTION

According to the present invention, it is possible to provide a gas adsorbent capable of suppressing desorption of gas components such as aldehyde, ammonia, and acetic acid adsorbed to an air filter from the air filter, that is, capable of suppressing secondary odor generation and deterioration over time of an aldehyde adsorbent, and having an effect of prolonging the life of formaldehyde gas removal performance.

EMBODIMENTS OF THE INVENTION

The present invention has achieved the above-described object, that is, as a result of intensive studies on providing a gas adsorbent capable of suppressing desorption of odor components such as aldehyde, ammonia, and acetic acid once adsorbed to an air filter from the air filter, that is, capable of prolonging the life of formaldehyde gas removal performance while suppressing secondary odor generation. Hereinafter, the present invention will be described in detail.

First, the gas adsorbent of the present invention contains an acid-impregnated activated carbon (A1), a base-impregnated activated carbon (A2), and an aldehyde adsorbent (B). Normally, activated carbon which is not impregnated with acid or base has only a so-called physical adsorption action of absorbing gas on the pore surface by an intermolecular force generated by contact with gas in the air due to its pore structure. Therefore, when the activated carbon adsorbs a gas exceeding the physical adsorption capacity of the activated carbon, the same components as those of the adsorbed gas are released again.

The acid-impregnated activated carbon (A1) is an activated carbon to which a so-called chemical adsorption action of changing a basic gas such as ammonia to a nonvolatile component different from that before adsorption by a neutralization reaction is imparted, in addition to the physical adsorption action inherent in the activated carbon, by impregnating an unimpregnated activated carbon with a chemical containing an acid to thereby load the acid on the pore surface of the activated carbon.

The acid of the acid-impregnated activated carbon (A1) used in the gas adsorbent of the present invention is not particularly limited. Examples thereof include inorganic acids such as phosphoric acid, hydrochloric acid, sulfuric acid, nitric acid and boric acid, and organic acids such as citric acid, oxalic acid and malic acid. Among them, an inorganic acid selected from phosphoric acid, hydrochloric acid, sulfuric acid, and nitric acid is more preferable from the viewpoint of the deodorizing effect.

The base-impregnated activated carbon (A2) is an activated carbon to which a so-called chemical adsorption action of changing an acidic gas such as acetic acid to a nonvolatile component different from that before adsorption by a neutralization reaction is imparted, in addition to the physical adsorption action inherent in the activated carbon, by impregnating an unimpregnated activated carbon with a chemical containing a base to thereby load the base on the pore surface of the activated carbon.

The base of the base-impregnated activated carbon (A2) used in the gas adsorbent of the present invention is not particularly limited. Examples thereof include hydroxides or salts of alkali metals and alkaline earth metal ions such as potassium, calcium, sodium, and magnesium. Specific examples thereof include potassium hydroxide, sodium hydroxide, potassium carbonate, sodium carbonate, potassium hydrogen carbonate, and sodium hydrogen carbonate. Among them, hydrogencarbonates such as potassium hydrogen carbonate and sodium hydrogen carbonate are preferable because pores of activated carbon are less likely to be blocked by a deliquescence phenomenon.

The method for impregnating activated carbon with an acid or base chemical is not particularly limited, but a method including spraying an aqueous solution or an aqueous dispersion of an acid or a base onto activated carbon, and performing drying to remove moisture, thereby fixing the chemical component to the pores of the activated carbon is preferable.

The acid-impregnated activated carbon (A1) and/or the base-impregnated activated carbon (A2) in the present invention preferably have the following pore structure. That is, at least one of the following conditions is preferably satisfied: the ratio of the pore volume of pores having a pore diameter of 0.4 to 2.0 nm of the acid-impregnated activated carbon (A1), calculated by the MP method to the pore volume of pores having a pore diameter of 0.4 nm or more of the acid-impregnated activated carbon (A1), calculated by the MP method and the BJH method is 75 to 100%; and the ratio of the pore volume of pores having a pore diameter of 0.4 to 2.0 nm of the base-impregnated activated carbon (A2), calculated by the MP method to the pore volume of pores having a pore diameter of 0.4 nm or more of the base-impregnated activated carbon (A2), calculated by the MP method and the BJH method is 75 to 100%.

As described above, among the pores of the acid-impregnated activated carbon (A1) and/or the base-impregnated activated carbon (A2), when the ratio of the pore volume of pores having a pore diameter of 0.4 to 2 nm to the pore volume of pores having a pore diameter of 0.4 nm or more is 75 to 100%, more preferably 80 to 100%, that is, the ratio of the pore volume of pores having a pore diameter of more than 2 nm to the pore volume of pores having a pore diameter of 0.4 nm or more is less than 25%, more preferably less than 20%, the adsorbent having this activated carbon is excellent in physical adsorption to an organic gas component such as toluene, and chemical adsorption performance for an acidic gas or a basic gas, and further, the re-release of the adsorbed gas component from the adsorbent is further suppressed.

The mechanism by which this effect is obtained is presumed as follows. That is, among the pores of the acid-impregnated activated carbon (A1) and the base-impregnated activated carbon (A2), the sizes of pores having a pore diameter of 0.4 to 2 nm, which are impregnated with an acid or a base, are close to the molecular size of an acidic gas such as acetic acid, or a basic gas such as ammonia. Therefore, such pores are excellent in physical adsorption performance, and further have characteristics that odor components once adsorbed in these pores are less likely to be desorbed from the pores. On the other hand, it is presumed that pores having a pore diameter of more than 2 nm are inferior in performance for suppressing desorption of odor components adsorbed in the pores (hereinafter, may be referred to as desorption suppression performance). In the acid-impregnated activated carbon (A1) and/or the base-impregnated activated carbon (A2) having a pore structure in which the ratio of the pore volume of pores having a pore diameter of 0.4 to 2 nm calculated by the MP method to the pore volume of pores having a pore diameter of 0.4 nm or more calculated by the MP method and the BJH method is 75 to 100%, the ratio of the amount of pores having an excellent desorption suppression performance for odor components to the amount of pores having a pore diameter of 0.4 nm or more present in these activated carbons is a specific value or more. In other words, the above-described pore structure is a pore structure in which the proportion of the total amount of pores having a poor desorption suppression performance for odor components is smaller than a specific value. As a result, it is presumed that the gas adsorbent including this activated carbon has an excellent desorption suppression performance for odor components.

Examples of the method for analyzing the pore diameter and the pore volume of the pore diameter of activated carbon include a micropore (MP) method and a Barrett-Joyner-Halenda (BJH) method. In the present invention, among the pores of the activated carbon, micropores having a pore diameter of 0.4 to 2 nm in which capillary condensation does not occur are analyzed by adopting the MP method. Further, in the present invention, among the pores of the activated carbon, macropores having a pore diameter of 2 nm or more are analyzed by adopting the BJH method. Here, the pore volume of pores having a pore diameter of 0.4 nm or more of activated carbon in the present invention, calculated by the MP method and the BJH method refers to the total value of the value of the pore volume obtained by the MP method and the value of the pore volume obtained by the BJH method.

The specific surface area of the acid-impregnated activated carbon (A1) and/or the base-impregnated activated carbon (A2) is preferably in a range of 400 to 1,300 $m^2/g$, more preferably in a range of 600 to 1,000 $m^2/g$. When the specific surface area of the acid-impregnated activated carbon and/or the base-impregnated activated carbon is within the above range, both the chemical adsorption performance of the chemical with respect to gas components in the air, and the physical adsorption performance of the pores of the activated carbon with respect to gas components in the air can be further achieved, which is preferable. The specific surface area as used herein can be measured according to the BET multipoint method (constant volume method described in 6.1 volumetric method, a measurement in accordance with a constant volume method, in which heating pretreatment is performed, and $N_2$ is used as an adsorbing substance) specified in JIS R 1626-1996. The impregnated amount of the chemical of the acid or base for obtaining the above-described specific surface area is preferably 2 to 40 mass%, more preferably 5 to 25 mass% with respect to the amount of the entire impregnated activated carbon.

The activated carbon having the pore structure as described above can be optionally selected from known materials such as coconut shell, charcoal, coal pitch, and phenol resin as a raw material. The activated carbon having such a pore structure can be obtained by adjusting activation conditions for forming pores by a high-temperature treatment with water vapor or a chemical treatment with phosphoric acid, zinc chloride, or the like. Among them, it is more preferable to select an activation method using coconut shell as a raw material and water vapor because activated carbon having a smaller pore size is easily obtained.

The aldehyde adsorbent (B) is a chemical adsorbent containing: inorganic particles as a carrier; and a chemical that has chemical reactivity with an aldehyde component such as acetaldehyde or formaldehyde, and that is attached to the support. As the inorganic particles, inorganic particles having a small reaction with the impregnated chemical are preferable.

The specific surface area of the inorganic particles employed in the present invention is preferably 50 to 1,200 $m^2/g$, more preferably 100 to 1,000 $m^2/g$. When the specific surface area of the inorganic particles is 50 to 1,200 $m^2/g$, the inorganic particles have mechanical strength, and a contact area effective for contact between the supported chemical adsorbent and the aldehyde gas is obtained and the reaction rate is improved, which is preferable. The specific surface area can be measured according to the BET multipoint method (constant volume method described in 6.1 volumetric method, a measurement in accordance with a constant volume method, in which heating pretreatment is performed, and $N_2$ is used as an adsorbing substance) specified in JIS R 1626-1996.

The pore volume of the inorganic particles is preferably 0.3 to 2.5 cc/g, more preferably 1.0 to 2.0 cc/g. When the pore volume of the inorganic particles is 0.3 to 2.5 cc/g, pores having excellent reactivity with an aldehyde gas in the air can be retained while the impregnated amount of the chemical agent is increased, thereby making it possible to increase adsorption efficiency as an aldehyde adsorbent and adsorption capacity, which is preferable. The pore volume as used herein can be measured by the BJH method described above.

As the inorganic particles, those having an average diameter of pores in the particles in a range of 0.5 to 100 nm are preferable, and 2 to 50 nm is more preferable. When the average diameter of the pores of the inorganic particles is to 0.5 to 100 nm, it is possible to increase the specific surface area for supporting the chemical while mechanical strength is secured even in a porous body, and the chemical easily permeates the inside of the pores, which is preferable. In addition, pores having a diameter of 2 to 50 nm are called mesopores, and particles having mesopores are excellent in efficiently advancing the reaction between the impregnated chemical and acetaldehyde. The average diameter of the pores as used herein can be calculated as the average pore diameter (D) from the specific surface area (S) and the pore volume (V) described above by the following formula. The shape of the pores is assumed to be cylindrical.

$$D \ = \ 4 \ V/S$$

The inorganic particles employed in the present invention can be selected, depending on the purpose, from among porous silicon dioxide (silica), zeolite, sepiolite, activated alumina, aluminum silicate, silica gel, alumina gel, activated clay, layered compounds such as zirconium phosphate and ammonium polytriphosphate, and porous clay minerals. Inorganic particles having a small reactivity with a chemical are preferable, and among them, porous silicon dioxide (silica) is preferable because it is possible to procure those having the above preferable average diameter of pores, specific surface area, and pore volume at low cost.

As the chemical having chemical reactivity with the aldehyde component employed in the present invention, primary to tertiary amine compounds can be used. Preferred examples thereof include hydrazide compounds such as adipic acid dihydrazide, dodecanedioic acid dihydrazide, and succinic acid dihydrazide, p-aminobenzenesulfonic acid, an ethyleneurea condensate chemical, and tris(hydroxymethyl)aminomethane. In particular, adipic acid dihydrazide is preferable from the viewpoint of adsorption performance for aldehydes. The use amount of adipic acid dihydrazide in the present invention is preferably 7 mg to 120 mg, more preferably 35 to 90 mg per 1 g of inorganic particles. When the use amount of adipic acid dihydrazide is within the above range, it is possible to obtain an aldehyde adsorbent excellent in the balance between the adsorption rate and the adsorption capacity of formaldehyde as a gas adsorbent.

There are two main reasons why the gas adsorbent of the present invention is composed of the above two types of chemical-impregnated activated carbons and an aldehyde adsorbent. First, when the gas adsorbent is used as an air filter, the gas in the air is composed of an extremely large number of components, and among them, a basic gas typified by ammonia, an acidic gas typified by acetic acid, and an aldehyde gas typified by acetaldehyde and formaldehyde have a low concentration threshold that humans feel as odor. As a means for suppressing re-release of these gases from the adsorbent, it is most effective to use all chemical adsorption with an acid, a base, and an aldehyde adsorbent, and to adsorb various gases other than acidic and basic gases by a physical adsorption action of activated carbon. On the other hand, when an acid and a base are impregnated into the same activated carbon, the respective effects are lost by the neutralization reaction of the acid and the base. Even if the acid and the base are respectively impregnated into different activated carbons, when the acid and the base are in contact with each other in one gas adsorbent, the neutralization reaction of the acid and the base also occurs, and the chemical adsorption performance is gradually deteriorated. Therefore, by interposing inorganic particles having no acidity or basicity between the acid and the base, it is possible to suppress the contact of the activated carbon with each of the acid and the base and to prolong the life of the chemical adsorption performance.

Second, many of the chemicals, which are impregnated into the inorganic particles and react with the aldehyde component, have high reactivity with the aldehyde gas in the air when the pH is from weakly acidic to near neutral. Therefore, an appropriate pH can be maintained by using the aldehyde adsorbent in combination with both the acid-impregnated activated carbon and the base-impregnated activated carbon, as compared with the case where the aldehyde adsorbent is used in combination with only one of the acid-impregnated activated carbon and the base-impregnated activated carbon. As a result, the aldehyde adsorption performance inherent in the chemical can be exhibited, and the performance can be maintained even after long-term storage.

The ratio $(M_A) : (M_B)$ of the total mass $(M_A)$ of the acid-impregnated activated carbon (A1) and the base-impregnated activated carbon (A2) to the mass $(M_B)$ of the aldehyde adsorbent (B) used in the gas adsorbent of the present invention is preferably in a range of 95 : 5 to 25 : 75, more preferably in a range of 90 : 10 to 35 : 65. By blending the gas adsorbent in this range, the re-release amount of the adsorbed gas when the gas adsorbent is used as a filter can be reduced, and deterioration in performance due to contact between the acid-impregnated activated carbon (A1) and the base-impregnated activated carbon (A2) can be suppressed. When the ratio of $(M_A)$ is 95 or less, it is possible to reduce the neutralization reaction caused by the contact between the acid-impregnated activated carbon (A1) and the base-impregnated activated carbon (A2) in the gas adsorbent, and to thereby suppress deterioration in the chemical adsorption performance of both carbons, which is preferable. Conversely, when the ratio of $(M_A)$ is 25 or more, the adsorption performance for acidic or basic gases in the air is more sufficient, and these gas components attached to the surface of the aldehyde adsorbent (B) are chemically adsorbed, and generation of unpleasant odor can be suppressed, which is preferable.

The pH of the gas adsorbent of the present invention is preferably 3 to 7, more preferably 5 to 7. When the pH is 3 to 7, the adsorption-desorption characteristics of the acidic gas, the basic gas, and the nonpolar gas such as formaldehyde are not biased, which is preferable. The pH of the gas adsorbent can be measured by adding 0.3 g of the gas adsorbent to 5 g of ultrapure water at a temperature of 20°C, and measuring the pH in water with a glass electrode pH meter.

The average particle diameter of the acid-impregnated activated carbon (A1) used in the gas adsorbent of the present invention is preferably 40 to 500 um, and the average particle diameter of the base-impregnated activated carbon (A2) is preferably 40 to 500 um. In addition, when the average particle diameter of the aldehyde adsorbent (B) is 100, the average particle diameter of the acid-impregnated activated carbon (A1) is preferably 95 to 600, and the average particle diameter of the base-impregnated activated carbon (A2) is preferably 95 to 600.

When the average particle diameter of the acid-impregnated activated carbon (A1) is 40 um or more, preferably 50 um or more, and the average particle diameter of the base-impregnated activated carbon (A2) is 40 um or more, preferably 50 um or more, the number of particles of the activated carbon can be appropriately suppressed, and the contact frequency between the acid-impregnated activated carbon (A1) and the base-impregnated activated carbon (A2) is reduced, so that deterioration in performance can be suppressed. When the average particle diameter of the acid-impregnated activated carbon (A1) is 500 um or less, preferably 400 um or less, and the average particle diameter of the base-impregnated activated carbon (A2) is 500 um or less, preferably 400 um or less, the degree of contact between one activated carbon particle and surrounding activated carbon particles can be reduced, so that deterioration in performance can be suppressed. The ratio between the average particle diameter of the acid-impregnated activated carbon (A1) and the average particle diameter of the base-impregnated activated carbon (A2) is preferably close. Specifically, when the average particle diameter of the acid-impregnated activated carbon (A1) is 100, the average particle diameter of the base-impregnated activated carbon (A2) is preferably 80 to 120.

Further, when the average particle diameter of the aldehyde adsorbent (B) is 100, the average particle diameter of the acid-impregnated activated carbon (A1) is 95 or more, preferably 100 or more, and the average particle diameter of the base-impregnated activated carbon (A2) is 95 or more, preferably 100 or more, the air permeability after the gas adsorbent is formed into a sheet shape can be made more sufficient. When the average particle diameter of the aldehyde adsorbent (B) is 100, the average particle diameter of the acid-impregnated activated carbon (A1) is 600 or less, preferably 500 or less, and the average particle diameter of the base-impregnated activated carbon (A2) is 600 or less, preferably 500 or less, the number of inorganic particles relative to the number of particles of the activated carbon is more appropriate. As a result, the inorganic particles can more sufficiently function as an intermediate particle that suppresses the contact between the acid-impregnated activated carbon (A1) and the base-impregnated activated carbon (A2).

Incidentally, the average particle diameter as used herein is obtained by measuring the particle diameter distribution according to JIS-Z-8815 (1994). The average particle diameter herein refers to a particle diameter corresponding to the size of a sieve mesh through which 50 mass% of the total mass of each of the particulate acid-impregnated activated carbon (A1), base-impregnated activated carbon (A2), and aldehyde adsorbent (B) is passed. In addition, the shapes of the acid-impregnated activated carbon (A1), the base-impregnated activated carbon (A2), and the aldehyde adsorbent (B) can be optionally selected from known shapes such as a spherical shape, a crushed shape, and a columnar shape.

The gas adsorbent of the present invention is preferably formed into a sheet shape and used. That is, the gas adsorbent of the present invention is suitably used for a gas adsorption sheet. Examples of the method for forming a gas adsorbent into a sheet shape as used herein include a method of forming a sheet shape by dispersing gas adsorbent particles between fibers of a fabric, and a method of forming a sheet shape by connecting surfaces of composite gas adsorbents with an adhesive or the like. Here, the form of the fabric is not particularly limited, and can be optionally selected from a woven fabric, a knitted fabric, a molded net, a nonwoven fabric, and the like. Among them, a nonwoven fabric is preferable because desired physical properties can be easily obtained by optionally selecting and combining the fiber diameter, fiber length, and the like of the fiber to be used. Examples of the nonwoven fabric include a chemical bond nonwoven fabric, a wet-laid certificate stamp nonwoven fabric, a spunbond nonwoven fabric, a meltblown nonwoven fabric, a spunlace nonwoven fabric, and an air-laid nonwoven fabric.

When the gas adsorbent of the present invention is formed into a sheet shape, the basis weight of the gas adsorbent is preferably in a range of 15 to 400 g/m$^2$. Further, when the basis weight is in a range of 30 to 300 g/m$^2$, the gas adsorption capacity is high, and the resulting sheet-shaped filter medium exhibits excellent pleating (folding) processability when being processed into an air filter, which is more preferable.

The gas adsorbent is preferably in the form of particles, and the shape of the particle can be optionally selected from known shapes such as a spherical shape, a crushed shape, and a columnar shape.

The gas adsorbent of the present invention is suitably used for a filter medium. The filter medium includes two or more layers of nonwoven fabric and the gas adsorbent of the present invention. The gas adsorbent of the present invention is held in at least one interface among one or more interfaces formed by two or more layers of nonwoven fabric.

The use amount of the gas adsorbent in the filter medium of the present invention is preferably in a range of 40 to 400 g/m$^2$, more preferably 100 to 200 g/m$^2$ from the viewpoint of obtaining gas removal efficiency and adsorption capacity when the gas adsorbent is used as a filter medium.

The nonwoven fabric included in the filter medium is preferably an electret nonwoven fabric. The electret nonwoven fabric is preferable because the filter medium can collect dust in the air with higher efficiency.

Specific examples of the method for producing the filter medium include, but are not limited thereto, a method in which certain amounts of gas adsorbent particles and powdery thermal adhesive resin particles are uniformly dispersed on one nonwoven fabric, the thermal adhesive resin particles are heated and melted by a heater, and then the other nonwoven fabric is layered thereon, and this is pressure-bonded for integration, and a method in which gas adsorbent particles are dispersed on one nonwoven fabric while a heated and melted resin is sprayed thereon, and then the other nonwoven fabric is layered thereon, and this is pressure-bonded for integration.

The thickness of the nonwoven fabric is preferably 0.08 to 0.60 mm, the lower limit thereof is more preferably 0.15 mm or more, and the upper limit thereof is more preferably 0.50 mm or less from the viewpoint of having a certain strength and increasing an area for which the nonwoven fabric can be accommodated in a certain volume at the time of pleating processing. The above-described filter medium has two or more layers of nonwoven fabrics, and the thicknesses of these nonwoven fabrics may be the same or different.

As the fibers used for the nonwoven fabric, natural fibers, synthetic fibers, or inorganic fibers such as glass fibers or metal fibers can be used. Among them, synthetic fibers made of a thermoplastic resin capable of melt spinning are preferable.

The air filter of the present invention includes the gas adsorption sheet of the present invention or the filter medium of the present invention, and an outer frame. It is preferable that four sides of the filter medium are fixed to the outer frame. Here, the gas adsorption sheet or the filter medium may be used in the form of a sheet or may form a three-dimensional shape having peaks and valleys in a pleated state.

EXAMPLES

Hereinafter, the operational effects of the present invention will be more specifically shown by Examples, but the present invention is not limited only to the following Examples.

[Method for producing filter medium using gas adsorbent]

A gas adsorbent and a polyethylene-based adhesive powder (Abifor 1200, manufactured by Abifor AG, hereinafter, referred to as adhesive powder) are blended at a mass ratio of 2 : 1 (gas adsorbent : adhesive powder). The blend is uniformly dispersed in a predetermined amount onto a spunbonded nonwoven fabric (AXTAR (registered trademark) H2070-1S, manufactured by Toray Industries, Inc., thickness: 0.27 mm) made of polyester fibers, and this is heated to 110°C to 130°C in a heating furnace to melt the adhesive powder. Then, an electret meltblown nonwoven fabric (basis weight: 30 g/m$^2$, thickness: 0.25 mm) is layered on the surface on which the blend was dispersed, and this is pressed with a nip roll to obtain a sheet-shaped filter medium having a predetermined thickness.

[Measurement methods]

(1) Saturated adsorption amount of toluene (g/m$^2$)

A sheet-shaped filter medium using the gas adsorbent obtained in the above [Method for producing filter medium using gas adsorbent] was collected into a square of 10 cm × 10 cm. This filter medium sample was placed in a dryer heated at 80°C, and dried for 2 hours, and the mass (g) of the collected filter medium was measured with an electronic balance. The obtained measured value was defined as m1 (g). Next, toluene was saturated in a 10 L desiccator with a humidity controlled to 50%RH, and the filter medium after mass measurement was placed in the desiccator and left for 24 hours. Then, the mass (g) of the filter medium taken out from the desiccator was measured with an electronic balance. The obtained measured value was defined as m2 (g). Next, the toluene saturated adsorption capacity per unit area of the filter medium was calculated from the following calculation formula.

· Toluene saturated adsorption capacity = (m2 - m1) / (0.1 × 0.1) (g/m$^2$).

(2) Desorption rate of toluene (%)

A circular filter medium sample (area: 28.3 cm$^2$) having a diameter of 6 cm was collected from a sheet-shaped filter medium using the gas adsorbent obtained in the above [Method for producing filter medium using gas adsorbent], and this filter medium sample was placed in a dryer heated at 80°C, dried for 2 hours, and then taken out. Next, two cylindrical wind tunnels having a ventilation diameter of 4 cm and a body length of 15 cm were prepared. Each of the two wind tunnels was attached to one surface side and the other surface side of the filter medium sample. Next, air containing a toluene gas having a concentration of 80 ppm, adjusted to a temperature of 20°C and a relative humidity of 50%, was allowed to pass at an air rate of 20 cm/sec in a direction from one surface side to the other surface side of the filter medium sample. Then, from 120 seconds after the toluene gas was allowed to pass through the filter medium sample, the toluene concentration (ppm) in the air on the downstream side of the filter medium sample (the other surface side of the filter medium sample) was measured at intervals of 10 seconds for 25 minutes using an infrared absorption gas concentration meter (MIR_AN SapphIRe, manufactured by Nippon Thermo Co., Ltd.). Then, the passing of the gas was stopped, and the integrated adsorption amount (g/m$^2$) of toluene per unit area (1 m$^2$) of the filter medium was calculated from the concentration of toluene detected in the air from the downstream side until that time.
After the measurement of the integrated adsorption amount of toluene, only air having a temperature of 20°C and a humidity of 50%RH was allowed to pass through the filter medium sample at an air rate of 0.06 m/sec. Then, from 20 seconds after the air was allowed to pass through the filter medium sample, the toluene concentration (ppm) in the air on the downstream side of the filter medium sample (the other surface side of the filter medium sample) was measured at intervals of 2 seconds for 5 minutes using the same infrared absorption gas concentration meter. The integrated desorption amount of toluene was measured from the concentration of toluene detected in the air. Then, the integrated desorption amount (g/m$^2$) of toluene per unit area (1 m$^2$) of the filter medium was calculated from the obtained measurement result.
The desorption rate (%) of toluene was calculated by the following formula from the obtained integrated adsorption amount (g/m$^2$) of toluene per unit area (1 m$^2$) and the obtained integrated desorption amount (g/m$^2$) of toluene per unit area (1 m$^2$).

Desorption rate of toluene (%) = integrated desorption amount (g/m$^2$) of toluene per unit area (1 m$^2$) / integrated adsorption amount (g/m$^2$) of toluene per unit area (1 m$^2$) × 100.

(3) Saturated adsorption amount of ammonia (g/m$^2$)

A circular filter medium sample (area: 28.3 cm$^2$) having a diameter of 6 cm was collected from a sheet-shaped filter medium using the gas adsorbent obtained in the above [Method for producing filter medium using gas adsorbent], and this filter medium sample was placed in a dryer heated at 80°C, dried for 2 hours, and then taken out. Next, two cylindrical wind tunnels having a ventilation diameter of 4 cm and a body length of 15 cm were prepared. Each of the two wind tunnels was attached to one surface side and the other surface side of the filter medium sample. Next, air containing an ammonia gas having a concentration of 10 ppm, adjusted to a temperature of 20°C and a relative humidity of 50%, was allowed pass at an air rate of 20 cm/sec in a direction from one surface side to the other surface side of the filter medium sample. Then, from 120 seconds after ammonia was allowed to pass through the filter medium sample, the ammonia concentration (ppm) in the air on the downstream side of the filter medium sample (the other surface side of the filter medium sample) was measured at intervals of 10 seconds for 60 minutes using an infrared absorption gas concentration meter (MIR_AN SapphlRe, manufactured by Nippon Thermo Co., Ltd.). The measurement was performed until the ammonia gas concentration on the downstream side of the sample reached 9 ppm, that is, until the ammonia adsorption efficiency of the filter medium reached 10%. The integrated adsorption amount ($g/m^2$) of ammonia per unit area ($1\ m^2$) of the filter medium was calculated from the obtained measurement result, and used as a saturated adsorption amount.

(4) Desorption rate of acetic acid (%)

A circular filter medium sample (area: $28.3\ cm^2$) having a diameter of 6 cm was collected from a sheet-shaped filter medium using the gas adsorbent obtained in the above [Method for producing filter medium using gas adsorbent], and this filter medium sample was placed in a dryer heated at 80°C, dried for 2 hours, and then taken out. Next, two cylindrical wind tunnels having a ventilation diameter of 4 cm and a body length of 15 cm were prepared. Each of the two wind tunnels was attached to one surface side and the other surface side of the filter medium sample. Next, air containing an acetic acid gas having a concentration of 80 ppm, adjusted to a temperature of 20°C and a relative humidity of 50%, was allowed to pass at an air rate of 20 cm/sec in a direction from one surface side to the other surface side of the filter medium sample. Then, from 120 seconds after the acetic acid gas was allowed to pass through the filter medium sample, the acetic acid concentration (ppm) in the air on the downstream side of the filter medium sample (the other surface side of the filter medium sample) was measured at intervals of 10 seconds for 25 minutes using an infrared absorption gas concentration meter (MIR_AN SapphlRe, manufactured by Nippon Thermo Co., Ltd.). Then, the passing of the gas was stopped, and the integrated adsorption amount ($g/m^2$) of acetic acid per unit area ($1\ m^2$) of the filter medium was calculated from the concentration of acetic acid detected in the air from the downstream side until that time.

After the measurement of the integrated adsorption amount of acetic acid, only air having a temperature of 20°C and a humidity of 50%RH was allowed to pass through the filter medium sample at an air rate of 0.06 m/sec. Then, from 20 seconds after the air was allowed to pass through the filter medium sample, the acetic acid concentration (ppm) in the air on the downstream side of the filter medium sample (the other surface side of the filter medium sample) was measured at intervals of 2 seconds for 5 minutes using the same infrared absorption gas concentration meter. The integrated desorption amount of acetic acid was measured from the concentration of acetic acid detected in the air. Then, the integrated desorption amount ($g/m^2$) of acetic acid per unit area ($1\ m^2$) of the filter medium was calculated from the obtained measurement result.

The desorption rate (%) of acetic acid was calculated by the following formula from the obtained integrated adsorption amount ($g/m^2$) of acetic acid per unit area ($1\ m^2$) and the obtained integrated desorption amount ($g/m^2$) of acetic acid per unit area ($1\ m^2$).

Desorption rate of acetic acid (%) = [integrated desorption amount ($g/m^2$) of acetic acid per unit area ($1\ m^2$) / integrated adsorption amount ($g/m^2$) of acetic acid per unit area ($1\ m^2$)] $\times$ 100.

(5) Formaldehyde clean air delivery rate (F-CADR) new ($m^3/hr$)

A sheet-shaped filter medium using the gas adsorbent obtained in the above [Method for producing filter medium using gas adsorbent] was prepared with a width of 289 mm and a length of 7.8 m. The filter medium was subjected to pleat processing to make 66 pleats each having a folding height of 58 mm in the length direction of the filter medium, using a reciprocating pleating machine (W650, manufactured by Hoptech Co., Ltd.), and then the filter medium was developed once. A polyolefin-based resin (Hi-Bon 9500, manufactured by Hitachi Kasei Polymer Co., Ltd.) melted by heating at 180°C using a hot melt applicator (Dynamelt, manufactured by ITW DynatecKK.) was continuously applied on each of the front and back surfaces of the filter medium, using a pressure nozzle such that total 6 lines of the melted resin were made with a thickness of 3 mm at intervals of 5 cm in the width direction of the filter medium. Then, the filter medium was folded again in accordance with the folds of the pleats, and the hot-melt resins applied in the line were bonded and fixed to each other such that the interval between adjacent pleat tips was 5.6 mm, thereby obtaining a pleated filter medium having a substantially rectangular parallelepiped shape.

An outer frame was obtained by cutting a polyester thermal bond nonwoven fabric having a basis weight of $260\ g/m^2$ and a thickness of 1 mm into a width of 60 mm. This outer frame was attached to four sides of the pleated filter medium using a polyolefin-based adhesive (Hi-Bon YH450-1, manufactured by Hitachi Kasei Polymer Co., Ltd.) which was melted by heating at 200°C by a roll coater (R2, manufactured by EPIC Co., Ltd.) to obtain an air filter having a length of 372 mm and a width of 291 mm (sizes in the front view), and a height of 60 mm.

The air filter was mounted on a commercially available air purifier (rated air volume: 450 m³/hr), and then placed in a test chamber having a volume of 30 m3. The CADR (m³/hr) for formaldehyde was measured by a method in accordance with "GB/T 18001-2015 Air cleaner".

(6) Formaldehyde clean air delivery rate (F-CADR) after long-term storage (m³/hr)

An air filter obtained in the same manner as in the above (5) was left under an environment of a temperature of 60°C and a relative humidity of 60%RH for 30 days. Then, the air filter was mounted on the same air purifier as the above (5), and a CADR (m³/hr) for formaldehyde was measured in the same test method as in the above (5).

(7) Odor intensity (point) and Comfort/ discomfort index (point) of secondary odor generation

An air filter obtained in the same manner as in the above (5) was mounted on a commercially available air purifier (rated air volume: 450 m³/hr). Next, this air purifier was placed in a transparent acrylic test chamber having a volume of 1 m3, and five pieces of tobacco (10 mg of MEVIUS) were burned in the test chamber. Then, the air purifier was operated to collect combustion smoke on the air filter for 30 minutes. This operation was repeated 10 times to collect tobacco combustion smoke of a total of 50 pieces.

The air purifier after the collection of tobacco combustion smoke was left for 24 hours without being operated in the test chamber having a volume of 30 m3, and the air purifier was then operated. The odor intensity and Comfort/ discomfort index of the discharged air were scored by five panelists according to the determination criteria shown in Tables 1 and 2, and the average value thereof was obtained.

[Table 1]

| Odor intensity | |
| --- | --- |
| **5** | Intense odor |
| **4** | Strong odor |
| **3** | Easily sensible odor |
| **2** | Weak odor that can be understood as to what odor |
| **1** | Finally sensed odor |
| **0** | Odorless |

[Table 2]

| Comfort/ discomfort index | |
| --- | --- |
| **-2** | Unpleasant |
| **-1** | Slightly unpleasant |
| **0** | Neither pleasant nor unpleasant |
| **1** | Slightly pleasant |
| **2** | Pleasant |

(8) Pore volume of pores of activated carbon

First, 0.10 g of activated carbon was placed in a glass cell, and the glass cell was degassed at 150°C for 5 hours. Thereafter, the activated carbon was charged into an apparatus BELSORP-18PLUS manufactured by BEL Japan Inc, under the conditions of a liquid nitrogen temperature of 77 K, an apparatus internal temperature of 35°C, and a saturated vapor pressure of 101.3 kPa. The isothermal adsorption and desorption processes of nitrogen were measured. From the measurement results, the pore volume of pores having a pore diameter of 0.4 to 2 nm was calculated by the MP method, and the pore volume of pores having a pore diameter of 0.4 nm or more was calculated by the BJH method.

[Example 1]

A gas adsorbent was used which was obtained by blending, as the acid-impregnated activated carbon, an impregnated activated carbon having a specific surface area of 450 m$^2$/g, obtained by impregnating a particulate activated carbon (MNC30/60-O, manufactured by Man-ei Kougyo, average particle diameter measured according to the JISZ8815 method: 300 um, specific surface area measured by the BET multipoint method: 1,200 m$^2$/g) with citric acid in an amount of 40 mass% of the whole, as the base-impregnated activated carbon, an impregnated activated carbon having a specific surface area of 800 m$^2$/g, obtained by impregnating the same particulate activated carbon as the above-described impregnated activated carbon with potassium carbonate in an amount of 7 mass% of the whole, and as the aldehyde adsorbent, an adsorbent obtained by impregnating porous silica particles (pore volume measured by the BJH method: 1.0 cc/g, specific surface area measured by the BET multipoint method: 500 m$^2$/g, average particle diameter measured according to the JISZ8815 method: 400 um) with adipic acid dihydrazide (manufactured by Nihon Kasei Co., Ltd.) in an amount of 7 mass% of the whole, at a mass ratio of 10 : 10 : 80 (acid-impregnated activated carbon : base-impregnated activated carbon : aldehyde adsorbent). Using this gas adsorbent, a filter medium having a gas adsorbent was produced by the method described in the above [Method for producing filter medium using gas adsorbent]. Since 100 g/m$^2$ of the gas adsorbent was used, the amount of the acid-impregnated activated carbon was 10 g/m$^2$, the amount of the base-impregnated activated carbon was 10 g/m$^2$, and the amount of the aldehyde adsorbent was 80 g/m$^2$. Thus, the mass ratio of the total of the impregnated activated carbons to the aldehyde adsorbent was 20 : 80. The thickness of the filter medium was 0.9 mm.
The measurements of the above (1) to (8) were performed using the obtained filter medium.

[Example 2]

A gas adsorbent obtained by blending the same acid-impregnated activated carbon, base-impregnated activated carbon, and aldehyde adsorbent as in Example 1 at a mass ratio of 30 : 30 : 90 (acid-impregnated activated carbon : base-impregnated activated carbon : aldehyde adsorbent) was used. Using this gas adsorbent, a filter medium having a gas adsorbent was produced by the method described in the above [Method for producing filter medium using gas adsorbent]. Since 150 g/m$^2$ of the gas adsorbent was used, the amount of the acid-impregnated activated carbon was 30 g/m$^2$, the amount of the base-impregnated activated carbon was 30 g/m$^2$, and the amount of the aldehyde adsorbent was 90 g/m$^2$. Thus, the mass ratio of the total of the impregnated activated carbons to the aldehyde adsorbent was 40 : 60. The thickness of the filter medium was 1.1 mm.
The measurements of the above (1) to (8) were performed using the obtained filter medium.

[Example 3]

A gas adsorbent obtained by blending the same acid-impregnated activated carbon, base-impregnated activated carbon, and aldehyde adsorbent as in Example 1 at a mass ratio of 36 : 36 : 8 (acid-impregnated activated carbon : base-impregnated activated carbon : aldehyde adsorbent) was used. Using this gas adsorbent, a filter medium having a gas adsorbent was produced by the method described in the above [Method for producing filter medium using gas adsorbent]. Since 80 g/m$^2$ of the gas adsorbent was used, the amount of the acid-impregnated activated carbon was 36 g/m$^2$, the amount of the base-impregnated activated carbon was 36 g/m$^2$, and the amount of the aldehyde adsorbent was 8 g/m$^2$. Thus, the mass ratio of the total of the impregnated activated carbons to the aldehyde adsorbent was 90 : 10. The thickness of the filter medium was 0.7 mm.
The measurements of the above (1) to (8) were performed using the obtained filter medium.

[Example 4]

A gas adsorbent was used which was obtained by blending, as the acid-impregnated activated carbon, an impregnated activated carbon having a specific surface area of 700 m$^2$/g, obtained by impregnating a particulate activated carbon (MNC30/60-O, manufactured by Man-ei Kougyo, average particle diameter measured according to the JISZ8815 method: 300 um, specific surface area measured by the BET multipoint method: 1,200 m$^2$/g) with orthophosphoric acid in an amount of 20 mass% of the whole, as the base-impregnated activated carbon, an impregnated activated carbon having a specific surface area of 950 m$^2$/g, obtained by impregnating the same particulate activated carbon as the above-described impregnated activated carbon with sodium hydrogen carbonate in an amount of 5 mass% of the whole, and as the aldehyde adsorbent, the same adsorbent as in Example 1, at a mass ratio of 30 : 30 : 90 (acid-impregnated activated carbon : base-impregnated activated carbon : aldehyde adsorbent). Using this gas adsorbent, a filter medium having a gas adsorbent was produced by the method described in the above [Method for producing filter medium using gas adsorbent]. Since 150 g/m$^2$ of the gas adsorbent was used, the amount of the acid-impregnated activated carbon was 30 g/m$^2$, the amount of the base-impregnated activated carbon was 30 g/m$^2$, and the amount of the aldehyde adsorbent was 90 g/m$^2$. Thus, the mass ratio of the total of the impregnated activated carbons to the aldehyde adsorbent was 40 : 60. The thickness of the filter medium was 1.2 mm.

The measurements of the above (1) to (8) were performed using the obtained filter medium.

[Example 5]

A gas adsorbent was used which was obtained by blending the same acid-impregnated activated carbon and base-impregnated activated carbon as in Example 4, and as the aldehyde adsorbent, an adsorbent obtained by impregnating porous silica particles (pore volume measured by the BJH method: 1.0 cc/g, specific surface area measured by the BET multipoint method: 500 m$^2$/g, average particle diameter measured according to the JISZ8815 method: 280 um) with adipic acid dihydrazide (manufactured by Nihon Kasei Co., Ltd.) in an amount of 7 mass% of the whole, at a mass ratio of 30 : 30 : 90 (acid-impregnated activated carbon : base-impregnated activated carbon : aldehyde adsorbent). Using this gas adsorbent, a filter medium having a gas adsorbent was produced by the method described in the above [Method for producing filter medium using gas adsorbent]. Since 150 g/m$^2$ of the gas adsorbent was used, the amount of the acid-impregnated activated carbon was 30 g/m$^2$, the amount of the base-impregnated activated carbon was 30 g/m$^2$, and the amount of the aldehyde adsorbent was 90 g/m$^2$. Thus, the mass ratio of the total of the impregnated activated carbons to the aldehyde adsorbent was 40 : 60. The thickness of the filter medium was 1.1 mm.

The measurements of the above (1) to (8) were performed using the obtained filter medium.

[Example 6]

A gas adsorbent was used which was obtained by blending, as the acid-impregnated activated carbon, an impregnated activated carbon having a specific surface area of 600 m$^2$/g, obtained by impregnating a particulate activated carbon (KURARAY COAL (registered trademark) GW30/60D, manufactured by Kuraray Co., Ltd., average particle diameter measured according to the JISZ8815 method: 300 um, specific surface area measured by the BET multipoint method: 950 m$^2$/g) with the same orthophosphoric acid as in Example 4 in an amount of 20 mass% of the whole, as the base-impregnated activated carbon, an impregnated activated carbon having a specific surface area of 850 m$^2$/g, obtained by impregnating the same particulate activated carbon as the above-described impregnated activated carbon with the same sodium hydrogen carbonate as in Example 4 in an amount of 5 mass% of the whole, and as the aldehyde adsorbent, the same adsorbent as in Example 5, at a mass ratio of 20 : 40 : 90 (acid-impregnated activated carbon: base-impregnated activated carbon: aldehyde adsorbent). Using this gas adsorbent, a filter medium having a gas adsorbent was produced by the method described in the above [Method for producing filter medium using gas adsorbent]. Since 150 g/m$^2$ of the gas adsorbent was used, the amount of the acid-impregnated activated carbon was 20 g/m$^2$, the amount of the base-impregnated activated carbon was 40 g/m$^2$, and the amount of the aldehyde adsorbent was 90 g/m$^2$. Thus, the mass ratio of the total of the impregnated activated carbons to the aldehyde adsorbent was 40 : 60. The thickness of the filter medium was 1.2 mm.

The measurements of the above (1) to (8) were performed using the obtained filter medium.

[Comparative Example 1]

A gas adsorbent obtained by blending the same citric acid-impregnated particulate activated carbon as in Example 1 as the acid-impregnated activated carbon and the same adsorbent as in Example 5 as the aldehyde adsorbent at a mass ratio of 60 : 90 (acid-impregnated activated carbon : aldehyde adsorbent) was used without using the base-impregnated activated carbon. Using 150 g/m$^2$ of this gas adsorbent, a filter medium having a gas adsorbent was produced by the method described in the above [Method for producing filter medium using gas adsorbent]. Since 150 g/m$^2$ of the gas adsorbent was used, the amount of the acid-impregnated activated carbon was 60 g/m$^2$, and the amount of the aldehyde adsorbent was 90 g/m$^2$. Thus, the mass ratio of the total amount of the impregnated activated carbons to the aldehyde adsorbent was 40 : 60. The thickness of the filter medium was 1.0 mm.

The measurements of the above (1) to (8) were performed using the obtained filter medium.

[Comparative Example 2]

A gas adsorbent obtained by blending the same potassium carbonate-impregnated particulate activated carbon as in Example 1 as the base-impregnated activated carbon and the same adsorbent as in Example 5 as the aldehyde adsorbent at a mass ratio of 60 : 90 (base-impregnated activated carbon : aldehyde adsorbent) was used without using the acid-impregnated activated carbon. Using 150 g/m$^2$ of this gas adsorbent, a filter medium having a gas adsorbent was produced by the method described in the above [Method for producing filter medium using gas adsorbent]. Since 150 g/m$^2$ of the gas adsorbent was used, the amount of the base-impregnated activated carbon was 60 g/m$^2$, and the amount of the aldehyde adsorbent was 90 g/m$^2$. Thus, the mass ratio of the total amount of the impregnated activated carbons to the aldehyde adsorbent was 40 : 60. The thickness of the filter medium was 1.0 mm.

The measurements of the above (1) to (8) were performed using the obtained filter medium.

[Comparative Example 3]

A gas adsorbent obtained by blending the same citric acid-impregnated particulate activated carbon as in Example 1 as the acid-impregnated activated carbon, and the same potassium carbonate-impregnated particulate activated carbon as in Example 1 as the base-impregnated activated carbon at a mass ratio of 50 : 100 (acid-impregnated activated carbon : base-impregnated activated carbon) was used without using the aldehyde adsorbent. Using a total of 150 g/m$^2$ of this gas adsorbent, a filter medium was produced in the same manner as in the method described in the above [Method for producing filter medium using gas adsorbent]. Since 150 g/m$^2$ of the gas adsorbent was used, the amount of the acid-impregnated activated carbon was 50 g/m$^2$, and the amount of the base-impregnated activated carbon was 100 g/m$^2$. Thus, the mass ratio of the total amount of the impregnated activated carbons to the aldehyde adsorbent was 100 : 0. The thickness of the filter medium was 1.0 mm.

The measurements of the above (1) to (8) were performed using the obtained filter medium.

Tables 3 and 4 summarize the gas adsorbents of Examples 1 to 6, and the filter mediums and air filters using the gas adsorbents of Examples 1 to 6. Tables 5 and 6 summarize the gas adsorbents of Comparative Examples 1 to 3, and the filter mediums and air filters using the gas adsorbents of Comparative Examples 1 to 3.

[Table 3-1]

| | | Example 1 | Example 2 | Example 3 |
|---|---|---|---|---|
| (A1) Acid-impregnated activated carbon | Acid | Citric acid 40 mass% | Citric acid 40 mass% | Citric acid 40 mass% |
| | (M1) mass (g/m$^2$) | 10 | 30 | 36 |
| | Average particle diameter (um) | 300 | 300 | 300 |
| | Specific surface area (m$^2$/g) | 450 | 450 | 450 |
| | (1) Pore volume of 0.4 to 2 nm pores (cc/g) (*1) | 0.35 | 0.35 | 0.35 |
| | (2) Pore volume of 2 to 200 nm pores (cc/g) (*2) | 0.15 | 0.15 | 0.15 |
| | (3) Total pore volume (cc/g) (1) + (2) | 0.50 | 0.50 | 0.50 |
| | (4) (1)/(3) × 100 (%) | 70 | 70 | 70 |
| (A2) Base-impregnated activated carbon | Base | Potassium carbonate 7 mass% | Potassium carbonate 7 mass% | Potassium carbonate 7 mass% |
| | (M2) mass (g/m$^2$) | 10 | 30 | 36 |
| | Average particle diameter (um) | 300 | 300 | 300 |
| | Specific surface area (m$^2$/g) | 800 | 800 | 800 |
| | (1) Pore volume of 0.4 to 2 nm pores (cc/g) (*1) | 0.34 | 0.34 | 0.34 |
| | (2) Pore volume of 2 to 200 nm pores (cc/g) (*2) | 0.13 | 0.13 | 0.13 |
| | (3) Total pore volume (cc/g) (1) + (2) | 0.47 | 0.47 | 0.47 |
| | (4) (1)/(3) × 100 (%) | 72 | 72 | 72 |
| | (MA) (M1) + (M2) | 20 | 60 | 72 |
| (B) Aldehyde adsorbent | Component | Silica + adipic acid dihydrazide 7 mass% | Silica + adipic acid dihydrazide 7 mass% | Silica + adipic acid dihydrazide 7 mass% |
| | Mass (MB) (g/m$^2$) | 80 | 90 | 8 |
| | Average particle diameter (um) | 400 | 400 | 400 |

(continued)

| | Example 1 | Example 2 | Example 3 |
|---|---|---|---|
| (MA):(MB) | **20:80** | **40 : 60** | **90:10** |

*1: Pore volume of pores having a pore diameter of 0.4 to 2 nm calculated by the MP method
*2: Pore volume of pores having a pore diameter of 2 to 200 nm calculated by the BJH method

[Table 3-2]

| | | Example 4 | Example 5 | Example 6 |
|---|---|---|---|---|
| (A1) Acid-impregnated activated carbon | Acid | Orthophosphoric acid 20 mass% | Orthophosphoric acid 20 mass% | Orthophosphoric acid 20 mass% |
| | (M1) mass (g/m$^2$) | 30 | 30 | 20 |
| | Average particle diameter (um) | 300 | 300 | 300 |
| | Specific surface area (m$^2$/g) | 700 | 700 | 600 |
| | (1) Pore volume of 0.4 to 2 nm pores (cc/g) (*1) | 0.35 | 0.35 | 0.42 |
| | (2) Pore volume of 2 to 200 nm pores (cc/g) (* 2) | 0.15 | 0.15 | 0.06 |
| | (3) Total pore volume (cc/g) (1) + (2) | 0.50 | 0.50 | 0.48 |
| | (4) (1)/(3) × 100 (%) | 70 | 70 | 88 |
| (A2) Base-impregnated activated carbon | Base | Sodium hydrogen carbonate 5 mass% | Sodium hydrogen carbonate 5 mass% | Sodium hydrogen carbonate 5 mass% |
| | (M2) mass (gm$^2$) | 30 | 30 | 40 |
| | Average particle diameter (um) | 300 | 300 | 300 |
| | Specific surface area (m$^2$/g) | 950 | 950 | 850 |
| | (1) Pore volume of 0.4 to 2 nm pores (cc/g) (*1) | 0.36 | 0.36 | 0.42 |
| | (2) Pore volume of 2 to 200 nm pores (cc/g) (* 2) | 0.13 | 0.13 | 0.07 |
| | (3) Total pore volume (cc/g) (1) + (2) | 0.49 | 0.49 | 0.49 |
| | (4) (1)/(3) × 100 (%) | 73 | 73 | 86 |
| (MA) (M1) + (M2) | | 60 | 60 | 60 |
| (B) Aldehyde adsorbent | Component | Silica + adipic acid dihydrazide 7 mass% | Silica + adipic acid dihydrazide 7 mass% | Silica + adipic acid dihydrazide 7 mass% |
| | Mass (MB) (g/m$^2$) | 90 | 90 | 90 |
| | Average particle diameter (um) | 400 | 280 | 280 |
| (MA): (MB) | | 40 : 60 | 40 : 60 | 40 : 60 |

*1: Pore volume of pores having a pore diameter of 0.4 to 2 nm calculated by the MP method
*2: Pore volume of pores having a pore diameter of 2 to 200 nm calculated by the BJH method

[Table 4]

| | Example 1 | Example 2 | Example 3 | Example 4 | Example 5 | Example 6 |
|---|---|---|---|---|---|---|
| Saturated adsorption amount of toluene (g/m$^2$) | 13 | 38 | 45 | 48 | 44 | 45 |
| Desorption rate of toluene (%) | 20 | 20 | 22 | 18 | 18 | 16 |
| Saturated adsorption amount of ammonia (g/m$^2$) | 0.30 | 0.45 | 0.60 | 0.75 | 0.75 | 0.75 |
| Desorption rate of acetic acid (%) | 17 | 16 | 14 | 14 | 14 | 12 |
| Formaldehyde CADR (m$^3$/hr) New | 180 | 200 | 150 | 200 | 210 | 200 |
| Formaldehyde CADR (m$^3$/hr) After long-term storage | 160 | 180 | 135 | 180 | 210 | 200 |
| Secondary odor generation Odor intensity (point) | 2.5 | 2.3 | 2.5 | 2.1 | 2.0 | 1.9 |
| Secondary odor generation Comfort/ discomfort index (point) | -0.5 | -0.5 | -0.5 | -0.4 | -0.4 | 0.3 |

EP 4 410 418 A1

[Table 5]

| | | Comparative Example 1 | Comparative Example 2 | Comparative Example 3 |
|---|---|---|---|---|
| (A1) Acid-impregnated activated carbon | Acid | Citric acid 40 mass% | None | Citric acid 40 mass% |
| | (M1) mass (g/m$^2$) | 60 | - | 50 |
| | Average particle diameter ($\mu$m) | 300 | - | 300 |
| | Specific surface area (m$^2$/g) | 450 | - | 450 |
| | (1) Pore volume of 0.4 to 2 nm pores (cc/g) (*1) | 0.35 | - | 0.35 |
| | (2) Pore volume of 2 to 200 nm pores (cc/g) (* 2) | 0.15 | - | 0.15 |
| | (3) Total pore volume (cc/g) (1) + (2) | 0.50 | - | 0.50 |
| | (4) (1)/(3) $\times$ 100 (%) | 70 | - | 70 |
| (A2) Base-impregnated activated carbon | Base | None | Potassium carbonate 7 mass% | Potassium carbonate 7 mass% |
| | (M2) mass (g/m$^2$) | - | 60 | 100 |
| | Average particle diameter ($\mu$m) | - | 300 | 300 |
| | Specific surface area (m$^2$/g) | - | 800 | 800 |
| | (1) Pore volume of 0.4 to 2 nm pores (cc/g) (*1) | - | 0.34 | 0.34 |
| | (2) Pore volume of 2 to 200 nm pores (cc/g) (* 2) | - | 0.13 | 0.13 |
| | (3) Total pore volume (cc/g) (1) + (2) | - | 0.47 | 0.47 |
| | (4) (1)/(3) $\times$ 100 (%) | - | 72 | 72 |
| (MA) (M1) + (M2) | | 60 | 60 | 150 |
| (B) Aldehyde adsorbent | Component | Silica + adipic acid dihydrazide 7 mass% | Silica + adipic acid dihydrazide 7 mass% | None |
| | Mass (MB) (g/m$^2$) | 90 | 90 | - |
| | Average particle diameter ($\mu$m) | 280 | 280 | - |

18

(continued)

|  | Comparative Example 1 | Comparative Example 2 | Comparative Example 3 |
|---|---|---|---|
| (MA):(MB) | **40:60** | **40:60** | **100:0** |
| *1: Pore volume of pores having a pore diameter of 0.4 to 2 nm calculated by the MP method<br>*2: Pore volume of pores having a pore diameter of 2 to 200 nm calculated by the BJH method | | | |

[Table 6]

| | Comparative Example 1 | Comparative Example 2 | Comparative Example 3 |
|---|---|---|---|
| Saturated adsorption amount of toluene (g/m$^2$) | 8 | 38 | 60 |
| Desorption rate of toluene (%) | 16 | 25 | 30 |
| Saturated adsorption amount of ammonia (g/m$^2$) | 0.68 | 0.05 | 0.60 |
| Desorption rate of acetic acid (%) | 40 | 16 | 25 |
| Formaldehyde CADR (m$^3$/hr) New | 200 | 80 | 40 |
| Formaldehyde CADR (m$^3$/hr) After long-term storage | 140 | 50 | 40 |
| Secondary odor generation Odor intensity (point) | 3.5 | 3.0 | 3.1 |
| Secondary odor generation Hedonic tone (point) | -1.0 | -1.5 | -1.5 |

In Example 1, a gas adsorbent obtained by combining an acid-impregnated activated carbon, a base-impregnated activated carbon, and an aldehyde adsorbent is used, and thus the desorption rate after adsorption is low. In addition, since citric acid used as an acid adsorbs ammonia gas in the air by an acid-base reaction, the adsorption capacity of ammonia is excellent, and since potassium carbonate used as a base adsorbs acetic acid gas in the air by an acid-base reaction, the desorption rate after acetic acid adsorption is low. Further, since the neutralization reaction due to the contact between citric acid and potassium carbonate, which were components impregnated into the activated carbon, was suppressed by the interposition of the inorganic particles, the adsorption performance for ammonia gas and acetic acid gas was maintained even after long-term storage. In addition, when the obtained filter medium was processed into a filter and mounted on an air purifier, the air filter had a good formaldehyde clean air delivery rate (F-CADR), a low odor intensity of secondary odor generation after adsorption of tobacco odor, and was less likely to generate odor that the user feels unpleasant, that is, had a long life.

In each of Examples 2 and 3, the use amounts of the acid-impregnated activated carbon and base-impregnated activated carbon, and the proportion of both activated carbons in the entire gas adsorbent were increased as compared with Example 1, and thus, the saturated adsorption amount of ammonia was increased, and the desorption rate after acetic acid adsorption was decreased as compared with Example 1. Therefore, when the obtained filter medium was processed into a filter and mounted on an air purifier, the air filter had a low odor intensity of secondary odor generation after adsorption of tobacco odor, and was less likely to generate odor that the user feels unpleasant, that is, had a long life.

In Example 4, an activated carbon which was impregnated with orthophosphoric acid having excellent reactivity with ammonia gas in the air than in Example 1 was used, and thus the saturated adsorption amount of ammonia was improved. In addition, by using sodium hydrogen carbonate which is less likely to block pores of the activated carbon as compared with Example 1, a base-impregnated activated carbon having a high physical adsorption was obtained, the desorption amount of acetic acid was low, and the saturated adsorption amount of toluene was improved. Therefore, when the obtained filter medium was processed into a filter and mounted on an air purifier, the air filter had a low odor intensity of secondary odor generation after adsorption of tobacco odor, and was less likely to generate odor that the user feels unpleasant, that is, had a long life.

In Example 5, an aldehyde adsorbent composed of inorganic particles having an average particle diameter substantially equal to the average particle diameters of the acid-impregnated activated carbon and the base-impregnated activated carbon was used. As a result, the neutralization reaction due to the contact between orthophosphoric acid and sodium hydrogen carbonate, which were components impregnated into the activated carbon, was suppressed by the interposition of the inorganic particles, and thus the adsorption performance for ammonia gas and acetic acid gas was maintained even after long-term storage. In addition, when the obtained filter medium was processed into a filter and mounted on an air purifier, the air filter had a good formaldehyde clean air delivery rate (F-CADR), a low odor intensity of secondary odor generation after adsorption of tobacco odor, and was less likely to generate odor that the user feels unpleasant, that is, had a long life.

In Example 6, an activated carbon in which the proportion of the pore volume of pores having a pore diameter of 0.4 to 2 nm in the pore volume of all the pores of the acid-impregnated activated carbon and the base-impregnated activated carbon is higher than those of Examples 1 to 5 was used, and thus, the desorption rate after adsorption of toluene and acetic acid was reduced as compared to Examples 1 to 5. In addition, when the obtained filter medium was processed into a filter and mounted on an air purifier, the air filter had a good formaldehyde clean air delivery rate (F-CADR), a low odor intensity of secondary odor generation after adsorption of tobacco odor, and was less likely to generate odor that the user feels unpleasant, that is, had a long life.

In Comparative Example 1, the base-impregnated activated carbon was not contained, and thus all the acetic acid gas in the air was adsorbed by the physical adsorption characteristics of the activated carbon, and the desorption rate after adsorption was significantly high. Therefore, when the obtained filter medium was processed into a filter and mounted on an air purifier, the odor intensity of secondary odor generation after adsorption of tobacco odor was high, and highly unpleasant odor was felt.

In Comparative Example 2, the acid-impregnated activated carbon was not contained, and thus the saturated adsorption capacity of ammonia was significantly low. Therefore, when the obtained filter medium was processed into a filter and mounted on an air purifier, the odor intensity of secondary odor generation after adsorption of tobacco odor containing high concentration of ammonia component was high, and highly unpleasant odor was felt. In addition, it is considered that the pH of the entire gas adsorbent was changed to the alkali side due to the influence of the base-impregnated activated carbon, and the formaldehyde adsorption performance was deteriorated, and the formaldehyde clean air delivery rate (F-CADR) was deteriorated.

In Comparative Example 3, the aldehyde chemical adsorbent was not used, and thus the formaldehyde clean air delivery rate (F-CADR) when the obtained filter medium was processed into a filter and mounted on an air purifier was low. In addition, the acid-impregnated activated carbon and the base-impregnated activated carbon were in close contact with each other, and the adsorption performance for ammonia and acetic acid was deteriorated due to the neutralization reaction of the impregnated chemicals. As a result, the re-release amount of gas components after adsorption of tobacco odor when the obtained filter medium was processed into a filter and mounted on an air purifier was increased, the intensity of secondary odor generation was strong, and highly unpleasant odor was felt.

INDUSTRIAL APPLICABILITY

The gas adsorbent according to the present invention is mainly mounted on a household air purifier, and is used for an air filter and a filter medium for cleaning indoor air.

**Claims**

1. A gas adsorbent comprising:

    an acid-impregnated activated carbon (A1);
    a base-impregnated activated carbon (A2); and
    an aldehyde adsorbent (B).

2. The gas adsorbent according to claim 1, wherein a ratio $(M_A) : (M_B)$ of a total mass $(M_A)$ of the acid-impregnated activated carbon (A1) and the base-impregnated activated carbon (A2) to a mass $(M_B)$ of the aldehyde adsorbent (B) is 95 : 5 to 25 : 75.

3. The gas adsorbent according to claim 1 or 2, wherein

    an average particle diameter of the acid-impregnated activated carbon (A1) is 40 to 500 um,
    an average particle diameter of the base-impregnated activated carbon (A2) is 40 to 500 um, and
    when an average particle diameter of the aldehyde adsorbent (B) is 100, the average particle diameter of the acid-impregnated activated carbon (A1) is 95 to 600, and the average particle diameter of the base-impregnated activated carbon (A2) is 95 to 600.

4. The gas adsorbent according to any one of claims 1 to 3, wherein at least one of the following conditions are satisfied:

    a ratio of a pore volume of pores having a pore diameter of 0.4 to 2.0 nm of the acid-impregnated activated carbon (A1), calculated by an MP method to a pore volume of pores having a pore diameter of 0.4 nm or more of the acid-impregnated activated carbon (A1), calculated by an MP method and a BJH method is 75 to 100%; and
    a ratio of a pore volume of pores having a pore diameter of 0.4 to 2.0 nm of the base-impregnated activated carbon (A2), calculated by an MP method to a pore volume of pores having a pore diameter of 0.4 nm or more of the base-impregnated activated carbon (A2), calculated by an MP method and a BJH method is 75 to 100%.

5. The gas adsorbent according to any one of claims 1 to 4, wherein a specific surface area of the acid-impregnated activated carbon (A1) is 400 to 1,300 m$^2$/g, and/or a specific surface area of the base-impregnated activated carbon (A2) is 400 to 1,300 m$^2$/g.

6. A gas adsorption sheet comprising the gas adsorbent according to any one of claims 1 to 5.

7. A filter medium comprising:

two or more layers of nonwoven fabric; and
a gas adsorbent,
wherein
the gas adsorbent is the gas adsorbent according to any one of claims 1 to 5, and
the gas adsorbent is held in at least one interface among one or more interfaces formed by the two or more layers of nonwoven fabric.

8. An air filter comprising the filter medium according to claim 7.

## INTERNATIONAL SEARCH REPORT

International application No.

**PCT/JP2022/034719**

**A. CLASSIFICATION OF SUBJECT MATTER**

*B01J 20/22*(2006.01)i; *A61L 9/014*(2006.01)i; *B01D 39/14*(2006.01)i; *B01J 20/28*(2006.01)i; *C01B 32/354*(2017.01)i; *C01B 33/18*(2006.01)i
FI:　B01J20/22 A; A61L9/014; B01D39/14 C; B01D39/14 N; B01J20/28 Z; C01B32/354; C01B33/18 E

According to International Patent Classification (IPC) or to both national classification and IPC

**B. FIELDS SEARCHED**

Minimum documentation searched (classification system followed by classification symbols)

B01J20/00-20/34; A61L9/014; B01D39/14-39/18; B01J20/28; C01B32/354-32/384; C01B33/18-33/193

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JSTChina/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | JP 2010-260045 A (TOYOTA BOSHOKU CORP) 18 November 2010 (2010-11-18) claims 1-4, paragraphs [0001], [0004], [0006]-[0055] | 1-6 |
| Y | | 7, 8 |
| X | JP 8-10315 A (MIDORI ANZEN CO LTD) 16 January 1996 (1996-01-16) claims 1-3, paragraphs [0001], [0003], [0010]-[0072] | 1-3, 6 |
| Y | | 7, 8 |
| A | JP 2017-64048 A (SUMINOE TEXTILE) 06 April 2017 (2017-04-06) entire text | 1-8 |

☐ Further documents are listed in the continuation of Box C.　　☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **11 October 2022** | **25 October 2022** |

| Name and mailing address of the ISA/JP | Authorized officer |
|---|---|
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (January 2015)

INTERNATIONAL SEARCH REPORT
Information on patent family members

| International application No.<br>**PCT/JP2022/034719** | | | |

| Patent document<br>cited in search report | Publication date<br>(day/month/year) | Patent family member(s) | Publication date<br>(day/month/year) |
|---|---|---|---|
| JP 2010-260045 A | 18 November 2010 | CN 101856890 A | |
| JP 8-10315 A | 16 January 1996 | (Family: none) | |
| JP 2017-64048 A | 06 April 2017 | (Family: none) | |

Form PCT/ISA/210 (patent family annex) (January 2015)

**REFERENCES CITED IN THE DESCRIPTION**

**Patent documents cited in the description**

- JP 2008148804 A
- JP 2016171875 A